(19) **Europäisches Patentamt**

**European Patent Office**

**Office européen des brevets**

(11) **EP 1 476 190 B1**

(12) **EUROPEAN PATENT SPECIFICATION**

(45) Date of publication and mention
of the grant of the patent:
**29.03.2006 Bulletin 2006/13**

(51) Int Cl.:
*A61K 41/00* (2006.01)   *A61P 35/00* (2006.01)
*A61P 31/18* (2006.01)   *A61K 31/22* (2006.01)

(21) Application number: **02701467.9**

(22) Date of filing: **29.01.2002**

(86) International application number:
**PCT/IB2002/000268**

(87) International publication number:
**WO 2003/063901 (07.08.2003 Gazette 2003/32)**

(54) **AMINO-SUBSTITUTED HYPOCRELLINS FOR USE AS SONOSENSITIZERS**

AMINOSUBSTITUIERTER HYPOCRELLINE ZUR VERWENDUNG ALS SONOSENSITIZER

HYPOCRELLINES AMINO-SUBSTITUEES UTILISABLES EN TANT QU'AGENTS
SONOSENSIBILISANTS

(84) Designated Contracting States:
**AT BE CH CY DE DK ES FI FR GB GR IE IT LI LU
MC NL PT SE TR**

(43) Date of publication of application:
**17.11.2004 Bulletin 2004/47**

(73) Proprietor: **Altachem Pharma, Ltd.**
**Edmonton, Alberta T6N 1E5 (CA)**

(72) Inventor: **MILLER, Gerald, G.**
**Montville, Queensland 4560 (AU)**

(74) Representative: **Brants, Johan P.E. et al**
**De Clercq, Brants & Partners cv**
**Edgard Gevaertdreef 10a**
**9830 Sint-Martens-Latem (BE)**

(56) References cited:
**WO-A-00/01413        WO-A-98/52609**
**WO-A-98/52610**

• **DIWU Z: "NOVEL THERAPEUTIC AND
DIAGNOSTIC APPLICATIONS OF
HYPOCRELLINS AND HYPERICINS"**
PHOTOCHEMISTRY AND PHOTOBIOLOGY,
OXFORD, GB, vol. 61, no. 6, 1995, pages 529-539,
XP000915971 ISSN: 0031-8655 cited in the
application

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

**Description**

Technical Field of the Invention

The invention involves compositions that are sonosensitizers for treating diseases. The compositions comprise amino-substituted hypocrellins, preferably demethoxylated hypocrellin B.

Background of the Invention

Treatment for cancer has traditionally encompassed three main strategies: surgery, chemotherapy, and radiotherapy. Although considerable progress in these areas has been attained, the search for more effective and safe alternative treatments continues. In this regard, photodynamic therapy (PDT) and sonodynamic therapy (SDT) represent a promising new approach(es) for the treatment of cancer. These therapies involve systemic or topical administration of a sensitizer, followed by its activation by light of a specific wavelength (PDT), or activation by sound of a specific frequency (SDT). The activation of the sensitizer leads to the production of activated oxygen and radical species that initiate a cascade of biochemical reactions, resulting in direct cell destruction, damage to the tumor vasculature and immune inflammatory responses. Lipson, et al. were the first to use photodynamic therapy, in 1966 at the Mayo Clinic [*Proc. IX Internat. Cancer Congress,* page 393 (1966)].

The photosensitizing and therapeutic properties of perylenequinonoid pigments (PQPs), such as hypocrellins, in biological systems have been recognized during the past two decades. See Diwu, et al., *J. Photochem. Photobiol. A: Chem.,* **64**:273 (1992); Zhang et al., (1989); and Wan, et al., "Hypocrellin A, a new drug for photochemotherapy," **Kexue Tongbao** (English edition) 26:1040 (1981). Because of the difficulty of collecting sufficient activated photosensitizer at the site of action, none of the previously known photosensitizers have gained widespread use as therapeutics.

The importance of sonodynamic therapy (SDT) lies ultimately in its similarity to PDT, an elegant and effective tumor treatment whose success is due to the use of light and drug in combination, i.e., two treatment elements, neither of which has toxic effects by itself (Marcus, 1992). The principal impetus for the development of SDT has been improvement upon PDT's dosimetric shortcomings. PDT is currently restricted to use with superficial tumors. Its use on tumors deep within the body requires interstitial irradiation that increases the complexity of the treatment and compromises its noninvasive nature. SDT provides a means to reach such tumors, since ultrasound propagates easily through several centimeters of tissue, and like light, can be focused principally on the tumor mass where it activates the sonosensitizing compound. Targeted SDT offers the possibility of improving the tolerance of this therapy by further restricting its effects to the target tissue.

While these discoveries represent significant advances, two serious deficiencies remain in the development of experimental SDT. A substantial problem is the lack of sonodynamic agents with favorable clinical properties. Porphyrins are known to cause significant cutaneous photosensitivity (Estey et al., 1996), doxorubicin is cardiotoxic (Myers et al., 1976), and DMSO, DMF and MMF are hepatotoxic (Misik and Riesz, 1996). New sensitizers with better sonodynamic properties, which have milder side effects and which are rapidly cleared, would greatly improve the clinical application of SDT. A further problem is the lack of standardization in the conditions used for evaluating sonodynamic agents.

Potential sonodynamic agents have been tested following exposure to ultrasound intensities ranging from $0.25W/cm^2$ to $40W/cm^2$, and frequencies from 500MHz to 1MHz (Harrison et al., 1991; Sasaki et al., 1998). Though *in vivo* use would seem to require greater energies due to roughly isotropic dissipation of the ultrasonic energy, little effort has been made to compare experimental conditions *in vitro* with those *in vivo.* Where one group will find evidence of sonodynamic effect, different investigators do not under apparently similar conditions. Development of standard insonation and assay systems compatible with clinical use will permit a more rigorous assessment of the sonodynamic effects of current and future sonosensitizers.

Sonodynamic activation of sensitizers has been found to be useful since ultrasound has the appropriate tissue attenuation coefficient for penetrating intervening tissues to reach desired treatment volumes, while retaining the ability to focus energy on reasonably small volumes. Diagnostic ultrasound is a well accepted, non-invasive procedure widely used in the developed world, and is considered safe even for fetal imaging. The frequency range of diagnostic ultrasound lies between 100 kHz -12 MHz, while 50 kHz sound provides enough energy to effect cellular destruction through microregional cavitation.

Sonodynamic therapy provides treatment strategies unavailable in standard photodynamic therapy, due to the limited tissue penetration of visible light. One example would be the treatment of newly diagnosed breast cancer, where local and regional spread of micrometastatic disease remains clinically undetectable. Using immunoconjugates (anti-breast cancer Mab - sonosensitizer hybrids), it would be theoretically possible to selectively eradicate micrometastases in the absence of normal tissue damage.

Beyond these basic properties shared with other waves, ultrasound exhibits unique properties when propagating through water. Above a certain threshold intensity, propagation of ultrasound waves through water elicits an effect termed 'cavitation' (Rayleigh, 1917; Connolly and Fox, 1954). Cavitation involves the formation of small bubbles or 'cavities' in the water during the rarefaction half of the wave cycle, followed by the collapse of these bubbles during the compression half of the cycle (Putterman, 1995). Cavities focus the energy of the incident ultrasonic radiation by many orders of magnitude (Hiller et al., 1992). The consequence is that regions of cavitation in water are sites of extremely high temperature and pressure. Estimates of the temperatures generated in a collapsing cavity range from 5000K to $10^6$K (Suslick et al. 1986; Flint and Suslick, 1991; Misik and Riesz, 1995; Kaiser, 1995).

The biological effects of exposure to ultrasound are the result of its physical and chemical effects. The most obvious biological effects of ultrasound treatment stem from heating of the medium through which it passes. Such heating is exploited during physiotherapy to help heal injured tissues. (Lehmann et al., 1967; Patrick, 1966), and has been investigated as a possible modality for tumor treatment. This is due to the sensitivity of many tumours to hyperthermia, a state in which tissue temperatures are elevated above 42°C (Doss and McCabe, 1976; Marmor et al., 1979; Sculier and Klastersky, 1981; Bleehen, 1982; Hynynen and Lulu, 1990). Ultrasound has also been used in combination with radiation therapy to improve treatment response *in vivo* compared to radiotherapy alone (Clarke et al., 1970; Repacholi et al., 1971; Mitsumori et al., 1996). A principal danger in the use of ultrasound for therapeutic purposes is the formation of 'hotspots' due to regions of constructive interference and preferential absorption of ultrasonic energy by bone regions with low curvature radii[+] (Lehmann et al., 1967; Linke et al., 1973). These hotspots can cause serious damage to nearby tissues (Hill, 1968; Bruno et al., 1998).

As is the case of hematoporphyrin derivatives, natural PQPs do not themselves exhibit absorptivity longer than 600 nm, a characteristic that inherently predicts a decreased capability of activation as tissue depth increases beyond 3-5mm. This means that the natural PQPs are not sufficiently strong for photodynamic therapy, and this limits their photodynamic therapy applications.

Deficiencies of current porphyrin and PQP photosensitizers for photodynamic therapy have stimulated the development of a series of second generation compounds which have improved properties with respect to light absorption in the red spectral range, purity, pharmacokinetics, and reduced cutaneous photosensitivity. These deficiencies also lead to investigating other forms of activating the sensitizer, e.g., activation using sound waves.

It has been noted that some photosensitizing compounds also confer cytotoxicity when they are activated by ultrasound. Sonodynamic activation of photosensitizers (as used hereinafter, "sonosensitizers") is clinically relevant, since ultrasound has the appropriate tissue attenuation coefficient for penetrating intervening tissues to reach desired treatment volumes, while retaining the ability to focus energy on reasonably small volumes. Diagnostic ultrasound is a well accepted, non-invasive procedure widely used in the developed world, and is considered safe, even for fetal imaging. The frequency range of diagnostic ultrasound lies between 100 kHz -12 MHz, while 50 kHz sound will provide enough energy to effect cellular destruction through microregional cavitation. The molecular mechanisms of sonodynamic cytotoxicity are currently not well understood, however a role for singlet oxygen has been postulated. Sonodynamic effects of porphyrins and porphyrin analogs in cell culture and experimental animal tumors have been reported. Sonodynamic therapy provides treatment strategies unavailable in standard photodynamic therapy, due to the limited tissue penetration of red, visible light. The sensitizers under investigation include proprietary molecules for which this use has never been reported. Proof-of-concept has been shown for 7 perylenequinones, used to sonosensitize human promyelocytic leukemia (HL-60) cells *in vitro.*

The ultrasound generator is clinically licensed in Europe and North America for physiotherapy use. It is readily adapted to *in vitro* studies, and is used in its "off-the-shelf" configuration in cancer therapy. A wide variety of neoplastic diseases, as well as infectious diseases, autoimmune and hyperproliferative diseases, or any disease where targeted therapy is appropriate, could benefit from the sonodynamic approach.

Summary of the invention

In accordance with the present invention, hypocrellin derivatives, such as amino-substituted hypocrellins, including but not limited to demethoxylated hypocrellin B (DMHB), have sonosensitizing properties and may be used to treat diseases and other conditions. Moreover, the HB derivatives of the present invention may be conjugated to a delivery moiety to enhance the ability of the PQP derivative to target pre determined cells or structures *in vitro* or *in vivo.*

The compositions of the present invention, activated by sound, exhibit substantial absorption in the therapeutic frequencies of ultrasound; produce high singlet oxygen yield; can be produced in pure, monomeric form; may be derivatized to optimize properties of ultrasound activation, tissue biodistribution, and toxicity; and are rapidly excreted. They afford nuclear targeting by covalent attachment to DNA minor-groove binding agents, such as stapled lexotropins, to enhance sonotoxicity. They are not genotoxic. This trait is important in the context of treatment-related secondary malignancies.

Many PQP properties are summarized in Diwu, et al., *J. Photochem. Photobiol. A: Chem.,* **64**:273 (1992). Some perylenequinones are also potent inhibitors of certain viruses, particularly human immunodeficiency virus (HIV), and also the enzyme protein kinase C (PKC). Both anti-HIV and anti-PKC activities of certain PQPs are light-dependent, a phenomenon implicated in the photodynamic therapy of cancers [Diwu, et al., *Biochem. Pharmacol.,* **47**:373-389 (1994)]. The Diwu et al paper also discloses the successful conjugation of HB to a protein.

The sonosensitizing compounds of the present invention, when administered systemically, distribute throughout the body. Over a short period, ranging from hours to days, the compounds clear from normal tissues, but are selectively retained by rapidly proliferating cells (e.g., cancer cells or psoriasis lesions) for up to several days. The amino-substituted hypocrellins (e.g., DMHBs) of the present invention are inactive and non-toxic until activated, e.g., exposed to light in a specific wavelength range or to sound in a specific frequency range.

The compounds of the present invention are also beneficial therapeutically due to their dual selectivity. The compounds of the present invention are selective in their ability to preferentially localize the drug at the site of a predetermined target, such as a cancer cell, and they are selective in that precise delivery of light and/or sound can be confined to a specific area.

The compositions of the present invention, when administered *in vivo,* such as intravenously, distribute throughout the body. In subsequent hours, and sometimes days, the compositions containing at least one perylenequinone derivative begin to clear from normal tissues, but are selectively retained for up to several days by hyperproliferating cells, such as cancer cells. The HB remains inactive and non-toxic until it is activated. In accordance with the present invention, the HB may be activated by sound. The hyperproliferating cells, now containing or contacted with a HB compound, may be exposed to an activation source, e.g., sound of an appropriate frequency. Exposing the site containing the hyperproliferating cells with the activation source permits selective activation of the retained perylenequinone derivative, which in turn initiates local necrosis or apoptosis in the hyperproliferating cell tissue leading to cell death.

In combination with the delivery system according to the present invention, the compositions and methods of the present invention permit increased selectivity by preferential localization of the perylenequinone derivative at the site of the targeted cells, and permit increased selectivity by confining the activation source to a specific area, e.g., sound confined to a discrete area.

Brief Description of the Drawings

Figure 1 shows several structures for the demethoxylated HB compounds of the present invention, where $R_1$, $R_2$, $R_3$, $R_4$ are $OCH_3$ or $NHCH_2Ar$ (Ar are phenyl or pyridyl group), $NHCH(CH_2)_n$ (where $-CH(CH_2)_n$ are alicyclic group and n=3,4,5,6). 2-BA-2-DMHB is where $R_1$, $R_2$, $R_3$ are $OCH_3$, and $R_4$ is $NH(CH_2)_3CH_3$. Alternatively, $R_1$, $R_2$, $R_3$, $R_4$ may be $OCH_3$ or $NHCH_2(CH_2)_nAr$, wherein Ar is a phenyl, naphthyl, polycyclic aromatic or a heterocyclic moiety, and n is 0-12.

Figure 2 shows that tumor growth is inhibited by SDT with DMHB.

Modes For Carrying Out the Invention

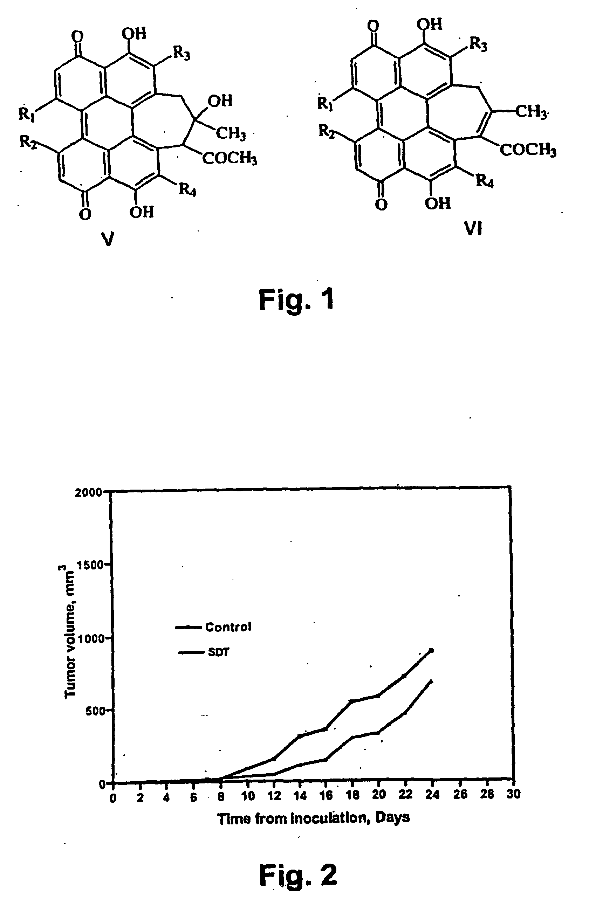

The present invention comprises the use of amino-substituted hypocrellins, e.g., the demethoxylated hypocrellin compounds of Figure 1, as sonodynamic agents, and the use of these compounds according to the invention as therapeutics.

The present invention includes a composition and method for manufacturing a composition for treating a pre-determined disease or condition comprising administering a therapeutic amount of a composition comprising a compound of the structure shown in Figure 1, allowing the compound to distribute to a portion of the body, preferably throughout the body, and activating the compound in an area containing hyperproliferating cells. In preferred embodiments of the invention, the activating step includes activating the DMHB compound with sound.

The present invention also includes compositions for use in a method that involves the topical application of a composition according to the invention, and activating the active agent in the composition. In preferred embodiments of the invention, the active agent is suitable for treating dermatological conditions, including but not limited to acne and hair removal.

The present invention also includes compositions for use in a method that involves the use of a composition according to the invention as an anti-bacterial agent in dental applications. In these embodiments of the invention, the active agent is formulated into a liquid composition, such as a mouthwash, contacting a tooth or teeth with the composition, and activating the active agent in the composition. In this embodiment of the invention, the composition is useful in treating cariotosis and the like. In preferred embodiments of the invention, the conjugate may be activated by sonoactivation.

The invention also comprises a composition for treating a disease by administering a therapeutically sufficient amount of at least one DMHB according to the invention, and activating the derivative(s) using sonoactivation. Typically, the DMHB compound may be activated by exposing the compound to a pre-determined sound frequency.

The invention also includes sonosensitive compounds that further comprise a cleavable linker, said linker being cleavable *in vivo.* In accordance with the present invention, the cleavable linker may be chosen to alter one or more properties of the compound, including but not limited to solubility, stability, absorption, and the like. Cleavable linkers include, but are not limited to, polyamides and sugars.

In a preferred embodiment of the invention, the HB is an amino-sustituted derivative of hypocrellin B. In the most preferred embodiments of the invention, the HB derivatives are demethoxylated hypocrellins (see Figure 1), where $R_1$, $R_2$, $R_3$, $R_4$ are $OCH_3$ or $NHCH_2Ar$ (Ar are phenyl or pyridyl group), $NHCH(CH_2)_n$ (where $-CH(CH_2)_n$ are alicyclic group and n=3,4,5,6). 2-BA-2-DMHB is where $R_1$, $R_2$, $R_3$ are $OCH_3$, and $R_4$ is $NH(CH_2)_3CH_3$. Altematively, $R_1$, $R_2$, $R_3$, $R_4$ may be $OCH_3$ or $NHCH_2(CH_2)_nAr$, wherein Ar is a phenyl, naphthyl, polycyclic aromatic or a heterocyclic moiety, and n is 0-12.

The compounds of the present invention may be produced by any method that results in a purified or substantially purified compound, or in a compound that is useful as a photodynamic or sonodynamic agent. The compounds of the present invention may also form a composition comprising a cocktail of compounds, e.g., more than one compound. These methods are well known in the art, e.g., Liu, et al., "Synthetic studies in novel hypocrellin B derivatives," **Tetrahedron, 49**:10785 (1993); and Diwu, et al., **Anti-Cancer Drug Design, 8**:129-143 (1993).

The amino-substituted HB compounds of the present invention are particularly suited for therapeutic use because they exhibit excellent sonodynamic activity in a frequency range from about 1MHz to about 3 MHz; are low molecular weight, typically from about 550 daltons to about 880 daltons); are available in pure monomeric form; exhibit rapid serum and skin clearance; have negligible cytotoxicity *in vitro* and *in vivo;* have excellent sonopotentiation (e.g., two orders of magnitude), so the safety margin in use is excellent; potent inhibitors of protein kinases; confer apoptotic cell death *in vitro* and *in vivo;* exhibit no genotoxicity; exhibit excellent tumor control; may be molecularly configured for targeted delivery; may be targeted to nuclear regions to further augment sonotoxicity; and the parent hypocrellins are amenable to site-specific modification, so that many derivatives may be formed, derivatives with varying degrees of sonosensitizing characteristics.

As used herein, "disease" refers to the management, diagnosis, and/or palliation of any mammalian (including human) disease, disorder, malady, or condition that can be treated by photodynamic therapy. "Disease" includes but is not limited to cancer and its metastases, such as skin cancer, growths or tumors, and their metastases; tumors and tumor cells, such as sarcomas and carcinomas, including solid tumors, blood-bome tumors, and tumors found in nasal passages, the bladder, the esophagus, or lung, including the bronchi ; viruses, including retroviruses; bacterial diseases; fungal diseases; and dermatological conditions or disorders, such as lesions of the vulva, keloid, vitiligo, psoriasis, benign tumors, endometriosis, Barett's esophagus, *Tinea capitis,* and lichen amyloidosis.

As used herein, "administering" and "delivering" refers to any action that results in exposing or contacting one or more DMHB derivatives with a predetermined cell, cells, or tissue, typically mammalian. As used herein, administering or delivering may be conducted *in vivo, in vitro,* or *ex vivo.* For example, a composition may be administered by injection or through an endoscope. Administering also includes the direct application to cells of a composition according to the present invention. For example, during the course of surgery, tumor cells may be exposed. In accordance with an embodiment of the invention, these exposed cells (or tumors) may be exposed directly to a composition of the present invention, e.g., by washing or irrigating the surgical site and/or the cells.

As used herein, activation, activating, or similar terms refers to the use of sound frequency to make a compound or portion of a compound more reactive. Any method for applying a sound source to a perylenequinone derivative may be used in accordance with the present invention, e.g., direct application, an ultrasound machine, focused ultrasound, high-intensity focused ultrasound, to name a few.

Upon application of the appropriate sound, the sensitizers can chemically (e.g., through oxidation, reduction and the like) change into a form that is toxic to the surrounding tissue. For example, following excitation of a sonosensitizer to a long-lived excited triplet state, a targeted tumor is destroyed either by the highly reactive singlet oxygen species (a Type II mechanism) and/or by free radical products (a Type I mechanism) generated by quantum energy transfer. Major biological target molecules of the singlet oxygen species and/or free radical products include nucleic acids, enzymes and cell membranes. A secondary therapeutic effect of the present methods involves the release of pathophysiologic products, such as prostaglandins, thromboxanes and leukotrienes, by tissue exposed to the effects of activated photosensitizers. Thus, it will be apparent to one skilled in the art that careful targeting of the sonoactive agents is of paramount importance to achieve therapeutic effects without eliciting toxemias.

As used herein, "sonosensitizing potential" refers to the property of the compound(s) to exert sound-mediated toxicity in excess of its (their) inherent unactivated toxicity. In a preferred embodiment of the invention, the activation factor may be calculated as the ratio of the $LD_{50}$ of cells treated without activation to the $LD_{50}$ of the cells treated with an activated compound (drug $LD_{50}$ divided by activated drug $LD_{50}$). Where the term "$LD_{50}$" has been used above, the term "$IC_{50}$" may be substituted, to address the bioassays that concern metabolic activity rather than the endpoint of lethality, loss of reproductive capability, or clonogenic death.

In accordance with the present invention, a desirable compound of the present invention is one that is non-toxic (or of low toxicity) at high drug concentrations without activation, i.e., without sound, and is toxic at low concentrations when sound of the appropriate frequency, is applied. As is recognized by those skilled in the art, the most desirable compounds are those that provide a wide range of non-toxic doses in an unactivated state, as this characteristic provides an increased safety factor for the patient.

As used herein, physiologically acceptable fluid refers to any fluid or additive suitable for combination with a composition containing a PQP derivative. Typically these fluids are used as a diluent or carrier. Exemplary physiologically acceptable fluids include but are not limited to preservative solutions, saline solution, an isotonic (about 0.9%) saline solution, or about a 5% albumin solution or suspension. It is intended that the present invention is not to be limited by the type of physiologically acceptable fluid used. The composition may also include pharmaceutically acceptable carriers. Pharmaceutically accepted carriers include but are not limited to saline, sterile water, phosphate buffered saline, and the like. Other buffering agents, dispersing agents, and inert non-toxic substances suitable for delivery to a patient may be included in the compositions of the present invention. The compositions may be solutions, suspensions or any appropriate formulation suitable for administration, and are typically sterile and free of undesirable particulate matter. The compositions may be sterilized by conventional sterilization techniques.

The composition containing a sonosensitizer of the present invention may be administered to the patient by any biologically suitable route. For example, the sonosensitizer may be introduced into the patient by intravenous, subcutaneous, intraperitoneal, intrathecal, intravesical, intradermal, intramuscular, or intralymphatic routes. The composition may be in solution, tablet, aerosol, or multi-phase formulation forms. Liposomes, long-circulating liposomes, immunoliposomes, biodegradable microspheres, micelles, or the like may also be used as a carrier, vehicle, or delivery system. Furthermore, using *ex vivo* procedures well known in the art, blood or serum from the patient may be removed from the patient; optionally, it may be desirable to purify the antigen in the patient's blood; the blood or serum may then be mixed with a composition that includes a sonosensitizer according to the invention; and the treated blood or serum is returned to the patient. The invention should not be limited to any particular method of introducing the sonosensitizer into the patient.

The amino-substituted HB derivatives of the present invention may also be used in conjunction with and conjugated to a number of other compounds, signaling agents, enhancers, and/or targeting agents. For example, a hypocrellin derivative of the present invention may be conjugated to an antibody, preferably a monoclonal antibody. In accordance with the present invention, the binding agent includes any DNA minor-groove targeting agent, such as lexotropsin or netropsin, preferably to enhance the toxicity through targeting the cell nucleus. Suitable enhancers include but are not limited to pKa modifiers, hypoxic cell radiosensitizers, and bioreductively activated anti-neoplastic agents, such as mitomycin C (preferably to effect or potentiate the toxicity of the compound in hypoxic cells or microorganisms). Suitable signaling agents include but are not limited to markers of apoptotic cell death or necrotic cell death, or regulatory molecules endogenous to cell cycle control or delay, preferably to potentiate the phototoxicity or sonotoxicity of the compound(s) by induction of apoptotic or necrotic cell death, or by inhibition of the repair of any form of lethal or potentially lethal damage (PLD).

In accordance with the present invention, the PQP derivatives may be functionalized, e.g., include reactive groups including but not limited to an acid, hydroxyl, an acid halide (preferably bromide), a hydrazine, a thiol, or a primary amine. The binding reagent may include reactive groups including but not limited to amino acids, such as cysteine, lysine, aspartic acid, glutamic acid and other dicarboxylic acid amino acids, and other tri- or poly-functional amino acid derivatives.

In accordance with another embodiment of the invention, a composition of the present invention may be administered alone, or in combination (sequentially or in batch) with other immunotherapeutic compositions. The sonosensitizers of the present invention may be further combined with agents for other activation modalities, e.g., photosensitizers and/or radiation sensitive agents. These features afford potential augmentation of the sonodynamic therapeutic ratio through sequential sensitizer administration (followed by sound activation). Under these conditions, a distant metastasis may be targeted.

In this embodiment of the invention, a SDT method comprises administering a first sonodynamic agent, preferably having a slow uptake, and administering a second sonodynamic agent, preferably having a more rapid uptake than that of the first agent. Both first and second sonodynamic agents may then be activated by exposing the patient and/or the agent to sound of suitable frequency, as described above.

The excellent fluorescence properties of the hypocrellins and derivatives provide a valuable tool to monitor intracellular uptake and distribution kinetics by confocal laser scanning microscopy (CLSM). Each drug has unique properties of uptake and distribution (Miller et al 1995 a,b). The rate cells take up drug in humans *in vitro* and *in vivo* can be determined using similar protocols as Liu et al 1995 and Miller et al., 1995 a or b).

Some of the embodiments of the present invention also have the added benefit of functioning with or without the presence of oxygen. Therefore, some embodiments of the present invention are effective in the treatment of solid tumors which are either well oxygenated or either partially or fully hypoxic.

The sono-activating agents may be formulated for topical application in penetrating solvents or in the form of a lotion, cream, ointment or gel containing a sufficient amount of the sonosensitizing agent compound to be effective for SDT therapy. Such topical formulations may be prepared in gel form by combining the photosensitizing agent with a solvent and adding a gelling agent thereto. Suitable gelling agents include carboxymethyl cellulose (Carbopol.TM. 934P from B. F. Goodrich of Brecksville, Ohio U.S.A.) and fumed silica (CAB-O-SIL.RTM., Cabot Corp., Tuscola, III.). The gelling agent is generally used in amounts of about 5-10 wt % to obtain a gel with the desired viscosity. Obviously, gels containing more or less gelling agent will have slightly higher or lower viscosity. One skilled in the art can readily obtain the desired gel viscosity by adjusting the concentration of gelling agent.

Additives, such as cosolvents, surfactants and/or bioadhesives frequently improve the gel properties and may be added as desired. Suitable cosolvents/surfactants include propylene glycol and glycerine. Suitable bioadhesives include carboxymethylcellulose, polyacrylic polymers, chitosan and sodium alginate, modified starch with polyacrylic polymers, eudispert hv hydrogels or xerogels, sodium hyaluronate, and polymers of polyethylene glycol, hydroxypropylcellulose, or carboxyvinyl. The additives may be incorporated into the gel by mechanically mixing the additives into a mixture of solvent and gelling agent.

Other additives may be used to enhance or maintain chemical stability and physiological suitability. Examples are antioxidants, chelating agents, inert gases, buffers and isotonicifiers. Examples of antioxidants and typical concentration ranges include acetone sodium bisulfite (0.1-0.8%), ascorbic acid (0.05-1.0%), monothioglycerol (0.1-1.0%), potassium metabisulfite (0.05-0.1%), propyl gallate (0.02%), sodium bisulfite (0.01-1.0%), sodium formaldehyde sulfoxylate (0.03-0.1%), sodium metabisulfite (0.02-0.25%), sodium sulfite (0.01-0.1%), sodium thioglycolate (0.05-0.1%).

Examples of chelating/complexing agents and typical concentration ranges include edetate sodium (0.005-0.1%), edetate calcium disodium (0.005%-0.01%), gentisic acid ethanolamide (1.0%-2.0%), niacinamide (1.0%-2.5%), sodium citrate (0.01%-2.5%), citric acid (0.001%-1.0%).

Buffers are used primarily to stabilize a formulation against the chemical degradation that might occur if the pH changed appreciably. Buffer systems employed normally have as low a buffer capacity as feasible in order to not disturb significantly the body buffer systems when injected. The buffer range and effect of the buffer on activity must be evaluated. Appropriate adjustment is useful to provide the optimum conditions for pH dependent partition into the target malignant tissues or lesion area. Examples of such buffer systems include the following acids: acetic, adipic, ascorbic, benzoic, citric, glycine, lactic, tartaric, hydrochloric, phosphoric, sulfuric, carbonic and bicarbonic; and their corresponding salts such as: potassium, sodium, magnesium, calcium and diethanolamine salts.

When the solution will be dispensed from multiple dose containers, antimicrobial agents in bacteriostatic or fungistatic concentrations are added in amounts effective to provide protection from bacteria or fungi. Among the compounds and concentrations most frequently employed are phenylmercuric acid (0.002-0.01%), thimerosal (0.01%), benzethonium chloride (0.01%), benzalkonium chloride (0.01%), phenol or cresol (0.5%), chlorbutanol (0.5%), benzyl alcohol (2.0%), methyl p-hydroxybenzoate (0.18%), propyl, p-hydroxybenzoate (0.02%), and ethylenediaminetetraacetic acid (EDTA).

Suitable penetrating solvents are solvents for the porphycene compound which will enhance percutaneous penetration of the porphycene compound. Solvents which have this property include proparacaine, dimethyl sulfoxide, dimethyl acetamide, dimethylformamide, 1-methyl-2-pyrrolidone, diisopropyladipate, diethyltoluamide and to a lesser extent propylene glycol. Additional solvents include substituted azacycloalkan-2-ones having from 5 to 7 carbons in the cycloalkyl group such as 1-dodecylazacycloheptan-2-one (AZONE) and other azacycloalkan-2-ones such as described in U.S. Pat. No. 3,989,816 incorporated herein by reference. Also included are N-bis-azocyclopentan-2-onyl alkanes described in U.S. Pat. No. 3,989,815, 1-substituted azacyclopentan-2-ones described in U.S. Pat. No. 3,991,203 (hereby incorporated by reference) and water-soluble tertiary amine oxides described in U.S. Pat. No. 4,411,893.

The sonosensitizing agents can be used with solvents and adjuvants appropriate to the sonosensitizing agent chemistry to adjust the viscosity of the formulation. The most important solvents in this group are ethanol, polyethylene - glycols of the liquid series and propylene glycol. A more comprehensive listing includes acetone, dimethyl acetamide, dimethyl formamide, dimethyl sulfoxide ethanol, glycerin, polyethylene glycol 300, and 400, propylene glycol, sorbitol, polyoxyethylene sorbitan fatty acid esters such as laureate, palmitate, stearate, and oleate, polyoxyethylated vegetable oil, sorbitan monopalmitate, 2-pyrrolidone; n-methyl-2-pyrrolidine; n-ethyl- 1 -pyrrolidine; tetrahydrofurfuryl alcohol, tween 80 and dimethyl isosorbide. Dimethyl isosorbide (ARLASOLVE®. DMI, ICI Specialty Chemicals) has the advantage of being both water- and oil-soluble. Additionally, dimethyl isosorbide may be readily gelled with a gelling agent to produce gel formulations with, for example, 4% KLUCEL.® (Hercules).

Additional topical formulations which may be used for the chosen sonosensitizing agent are disclosed in U.S. Pat. Nos. 3,592,930 and 4,017,615.

Examples

Example 1. Screening Assay for Efficiency of Sonosensitizers *in Vitro:*

This screening assay makes use of human promyelocytic leukemia cells growing in suspension culture. The transducer of an UltraMax ultrasound generator for physiotherapy was adapted for *in vitro* studies by fitting a tissue culture-compatible Teflon cuff such that cell suspensions could be conveniently insonated. The following diagrams represent the equipment employed.

Human acute promyelocytic leukemia HL-60 cells (ATCC No CCL-240) were maintained in RPMI 1640 cell culture medium supplemented with 7g/L of L-glutamine (Gibco, Grand Island NY), 10% fetal bovine serum (Cansera), and 1% penicillin-streptomycin-neomycin (Gibco), buffered with $NaHCO_3$ to pH 7.4. They were incubated at 37°C in a humidified atmosphere of 95% air, 5% $CO_2$, and maintained at cell densities between $2.0 \times 10^5$ to $1.0 \times 10^6$ cells/mL. For insonations in the presence of Hp (a porphyrin positive control) or hypocrellin test compounds, cells were brought to approximately $1.0 \times 10^6$ cells/mL, incubated with the putative sensitizer at 37°C for 2 hours in T-flasks, equilibrated for 30 minutes at room temperature, resuspended and insonated. All procedures took place in low - light conditions to avoid spurious photosensitization. Cells were gently resuspended for 10 seconds before insonation. The test sonosensitizer was dissolved in dimethylsulfoxide (DMSO) such that the final concentration of DMSO in the insonation medium did not exceed 1% (v/v). Stock sensitizer was prepared by slowly adding RPMI-1640 medium to the appropriate volume at final sensitizer concentrations between 0.3mM and 1mM.

Sonodynamic Therapy Survival Assay:

Aliquots of cells were withdrawn prior to insonation to serve as the original value for cell survival. Cells were suspended in Isoton diluent and counted in a Coulter electronic particle counter. Insonation proceeded at a power density of 2W, at times varying between 3- and 10 seconds to effect cytotoxicity by sonosensitization. Control cultures were subjected to the appropriate dose of ultrasound, in the presence of DMSO, and the absence of the test sonosensitizer. Following ultrasound treatment, aliquots of the cell suspension were withdrawn for counting as above, and the survival fraction, S, was calculated as the fraction:

$$S = (\text{Cell number following insonation/cell number prior to insonation}) \times 100.$$

Results:

We are currently interested in demethoxy hypocrellin B (DMHB) as a sonosensitizer, based on its excellent properties of photosensitization. Standard dose-response curves, with a fixed dose and energy of ultrasound demonstrate proof-of-concept that DMHB is an extremely efficient sonosensitizer of human cells *in vitro.* The $LD_{50}$ of the positive hematoporphyrin (Hp) control is 1.0mM, whereas the $LD_{50}$ for DMHB is approximately 100μM, a 10-fold difference. Conclusion: DMHB has high potential as an effective sonosensitizer *in vivo.*

|  | DMSO Only | DMSO + 1μM DMHB | DMSO + 10μM DMHB | DMSO + 100μM DMHB |
|---|---|---|---|---|
| Survival, % Mean | 55.8 | 41.8 | 37.3 | 30.7 |
|  | 53.0 | 48.2 | 43.3 | 39.4 |
|  | 40.0 | 33.5 | 24.9 | 23.7 |
|  | 49.6 | 41.1 | 35.2 | 31.3 |
| s.d. | 8.4 | 7.3 | 9.4 | 7.9 |
| Mean - DMSO Bg | 100.0 | 83.0 | 71.1 | 63.2 |

Example 2. Efficiency of DMHB *in Vivo:*

We explored the use of DMHB in Balb/c mice engrafted in the flank with the syngeneic murine mastocytoma, EMT6. The mice were inoculated subcutaneously with $1.0 \times 10^5$ EMT6 tumor cells in sterile injectable saline. Within 7 days, detectable tumors are evident. Mice were randomized into a control group and a SDT treatment group, which differed only in that the sonosensitizer was not present in the drug solvent (see below). Fresh DMHB was dissolved in mineral oil, and injected intraperitoneally into each mouse to achieve a total body concentration of 50μM. Following a an interval of 48h for biodistribution of the sensitizer, the mice were tightly anesthetized with Metafane, and placed on a 2% agarose gel, with the tumor lying in a depression of the gel which was juxtaposed to the transducer of an UltraMax physiotherapy ultrasound generator. Ultrasound was applied at a frequency of 1MHz, at a power density of $0.2W/cm^2$, for a 10-minute period. The maximum energy applied to the tumor volume was approximately 500J. Tumor growth was monitored daily by caliper measurements in three mutually orthogonal planes. Tumor volume was calculated from the expression,

$$V = \pi/6 \times d_1 \times d_2 \times d_3$$

where V = volume ($mm^3$), and $d_{1,2,}$ and $_3$ are the three mutually orthogonal diameters. The endpoint for the assay was the proportion of tumors reaching 4 times treatment volume, which was plotted as a function of time. Animals were euthanized at this point. Figure 2 is a plot of tumor growth vs. time.

Example 3.

HL-60 cells were treated with perylenequinone sensitizers and insonated as described above. The surviving fractions were plotted against sensitizer concentration. At a concentration of approximately 30μM, CPMg(Ac$_2$) showed sonotoxicity exceeding that of the 1000μM Hp control, with the decrease in survival occurring steeply over the preceding two decades of sensitizer concentration. DBHB and DMHB showed negligible sonotoxicity up to 100μM; the bulk of the observed sonotoxic effect occurred over the decade from 100μM to 1000μM, and the maximum effect was comparable to that of the Hp control. HBMg(Ac$_2$) showed no sonotoxic effect until 10μM. Cell survival decreased steeply over the next two decades of sensitizer concentration.

Example 4. Perylenequinonoid Pigments (Hypocrellins) and Their Photosensitizing and Sonosensitizing Properties

| Compound | | Photosensitizing Potential* | Sonosensitizing Potential* |
|---|---|---|---|
| **DMHBa** | Demethylated-HB | 3.0μM | 1.0mM |
| **DMHBb** | 2-butylamino-2-demethoxy-Hypocrellin B | 0.1μM | 0.1mM |
| **HA** | Hypocrellin A | 4.0μM | None |
| **HBAC-R1** | Cystamine-HB isomer 1 | 1.0μM | None |
| **HBAC-R2** | Cystamine-HB isomer 1 | 5.0μM | None |
| **HBAM-R1** | 2-morpholino-ethylaminated HB | 4.0μM | None |
| **HBDD-R1** | 2-(N,N-dimethyl-amino) propylamine-Hypocrellin B | | 1.0mM |
| **HBEA-R1** | Ethanolamine-Hypocrellin B isomer 1 | 0.15μM | 1.0mM |
| **HBEA-R2** | Ethanolamine-Hypocrellin B isomer 2 | 7.50μM | None |
| **HBED-R2** | Ethylenediamne-Hypocrellin B | 4.0μM | None |
| **HBMA-IV** | Methylamine-Hypocrellin B | 1.0μM | None |
| *Molar Concentration which exerts LD$_{50}$ in EMT6 murine mastocytoma *in vitro,* for a fixed dose of light or ultrasound | | | |

Example 5.

150 mg Hypocrellin B (0.28 mmol) is dissolved in 100 mL fresh distilled benzene containing excessive cyclopentylamine and the resulting solution is stirred for 18 hours. The solvent is removed under reduced pressure. Chloroform is used to wash the residue for 3-4 times, and the chloroform layer is washed with water for 3-4 times. Chloroform is evaporated, then thin-layer chromatography (TLC) is used to purify the residue using 3:1:0.5 (V:V:V) cyclohexane-ethyl acetate-95% ethanol as eluent. TLC is repeated twice for further purification, the amino-substituted demethoxylated hypocrellin B is obtained.

Characteristic of the product:

UV-vis spectra ($\lambda_{max}$): 466 nm, 577 nm, 641 nm;

IR spectra ($V_{max}$): 3419 cm$^{-1}$, 2922 cm$^{-1}$, 1680 cm$^{-1}$, 1608 cm$^{-1}$;

1HNMR ($\delta$): 6.32 (s), 6.80 (s), 3.08 (s), 2.55 (s), 1.79 (s), 1.91 (s), 4.01 (s), 4.08 (s), 4.24 (s), 1.86 (m), 1.68 (m), 1.49 (m), 1.00 (m), 16.07, 15.78 ppm;

Mass spectra (m/z): 583 (M$^+$)

Example 6.

Hypocrellin B (HB) was prepared by quantitative potassium hydroxide dehydration of hypocrellin A (HA) followed by neutralization with Ha, chloroform extraction, and recrystallization with benzene-petroleum ether, 2-butylamino-2-demethoxy-hypocrellin B (2-BA-2-DMHB) was prepared by reflux with n-butylamine in pyridine, neutralization, and chloroform extraction of HB. The product was subjected to 1 % citric acid-silica gel thin-layer chromatography (TLC), using a 6:1:1 mixture of petroleum ether/ethyl acetate/ethanol (95%) as eluent, and three compounds were obtained. They were the target compound (rate of flow (Rr) =0.64) and two by-products (Rr= 0.74 and 0:40. respectively), which were identified by satisfactory NMR, mass spectra and elemental analysis [12]. The target compound was further purified with TLC and the desired product, 2-BA-2-DMHB, was obtained in 54% yield. The purity or HB and 2-BA-2-DMHB was assessed by high-performance liquid chromatography and found to be higher than 95%. The chemical structure of 2-BA-2-DMHB is shown in Figure 1. The sonosensitizers were kept in lyophilized form until the day of experiments, at which time they were dissolved in DMSO.

While the invention has been described in some detail by way of illustration and example, it should be understood that the invention is susceptible to various modifications and alternative forms, and is not restricted to the specific embodiments set forth. It should be understood that these specific embodiments are not intended to limit the invention but, on the contrary, the intention is to cover all modifications, equivalents, and alternatives falling within the spirit and scope of the invention.

**Claims**

1. Use of an amino-substituted hypocrellin compound as a sonosensitizer for the preparation of a medicament for sonodynamic therapy.

2. Use of an amino-substituted hypocrellin compound as a sonosensitizer for the preparation of a medicament for the treatment by sound of skin conditions, cancer, viral diseases, retroviral diseases, bacterial diseases, autoimmune diseases or fungal diseases.

3. Use according to claim 1 or 2 wherein said amino-substituted hypocrellin compound is activatable by sound.

4. Use according to claim 3, wherein the activation is performed by a frequency of between about 50 kHz and about 12 MHz.

5. Use according to claim 4, wherein the frequency is between about 1 MHz and about 3 MHz.

6. Use according to any of the claims 1 to 5, wherein said amino-substituted hypocrellin compound is further activatable by light of a pre-determined wavelength.

7. Use according to any of the previous claims 1 to 6, wherein said compound is a demethoxylated hypocrellin B.

8. Use according to claim 7, wherein said demethoxylated hypocrellin B is 2-butylamino-2-demethoxy-hypocrellin B.

9. Use according to any of claims 1 to 8, of a composition comprising said sonosensitizer, and at least one of a pKa modifier, a buffer, a salt, a base, an acid, saline, and an adjuvant.

10. Use according to any of claims 1 to 9, of an amino-substituted hypocrellin compound for the preparation of a pharmaceutical composition for the sonodynamic treatment of skin conditions, cancer, viral diseases, retroviral diseases, bacterial diseases, autoimmune diseases or fungal diseases.

11. Use of an amino-substituted hypocrellin compound for the preparation of a sono-activatable pharmaceutical composition for treatment by sound of skin conditions, cancer, viral diseases, retroviral diseases, bacterial diseases, autoimmune diseases and fungal diseases.

**Patentansprüche**

1. Verwendung einer amino-substituierten Hypokrellinverbindung als Sonosensitizer für die Zubereitung eines Medikaments zur sonodynamischen Therapie.

2. Verwendung einer amino-substituierten Hypokrellinverbindung als Sonosensitizer für die Zubereitung eines Medikaments für die Behandlung von Krankheitszuständen der Haut, Krebs, viralen Erkrankungen, retroviralen Erkrankungen, bakteriellen Erkrankungen, Autoimmunerkrankungen, oder pilzbedingten Erkrankungen mittels Schall.

3. Verwendung nach Anspruch 1 oder 2, wobei die amino-substituierte Hypokrellinverbindung durch Schall aktivierbar ist.

4. Verwendung nach Anspruch 3, wobei die Aktivierung mittels einer Frequenz von zwischen ungefähr 50 kHz und ungefähr 12 MHz durchgeführt wird.

5. Verwendung nach Anspruch 4, wobei die Frequenz zwischen ungefähr 1 MHz und ungefähr 3 MHz ist.

6. Verwendung nach einem der Ansprüche 1 bis 5, wobei die amino-substituierte Hypokrellinverbindung darüber hinaus durch Licht einer vorbestimmten Wellenlänge aktivierbar ist.

7. Verwendung nach einem der vorhergehenden Ansprüche 1 bis 6, wobei die Verbindung ein demethoxyliertes Hypokrellin B ist.

8. Verwendung nach Anspruch 7, wobei das demethoxylierte Hypokrellin B 2-Butylamino-2-Demethoxy-Hypokrellin B ist.

9. Verwendung nach einem der Ansprüche 1 bis 8 von einer Zusammensetzung umfassend den Sonosensitizer, und wenigstens einen pKa Modifizierer, einen Puffer, ein Salz, eine Base, eine Säure, Salzlösung, und ein Adjuvans.

10. Verwendung nach einem der Ansprüche 1 bis 9 einer amino-substituierten Hypokrellinverbindung für die Zubereitung einer pharmazeutischen Zusammensetzung für die sonodynamische Behandlung von Krankheitszuständen der Haut, Krebs, viralen Erkrankungen, retroviralen Erkrankungen, bakteriellen Erkrankungen, Autoimmunerkrankungen oder pilzbedingten Erkrankungen.

11. Verwendung einer amino-substituierten Hypokrellinverbindung für die Zubereitung einer schallaktivierbaren pharmazeutischen Zusammensetzung für die Behandlung von Krankheitszuständen der Haut, Krebs, viralen Erkrankungen, retroviralen Erkrankungen, bakteriellen Erkrankungen, Autoimmunerkrankungen und pilzbedingten Erkrankungen mittels Schall.

**Revendications**

1. Utilisation d'un composé d'hypocrelline amino-substituée en tant qu'agent sonosensibilisant pour la préparation d'un médicament destiné à la thérapie par la dynamique sonore.

2. Utilisation d'un composé d'hyprocrelline amino-substituée en tant qu'agent sonosensibilisant pour la préparation d'un médicament destiné au traitement par le son des conditions dermiques, cancer, maladies virales, maladies rétrovirales, maladies bactériennes, maladies auto-immunes ou maladies fongiques.

3. Utilisation selon la revendication 1 ou 2, dans laquelle ledit composé d'hypocrelline amino-substituée peut être activé par le son.

4. Utilisation selon la revendication 3, dans laquelle l'activation s'effectue par une fréquence entre environ 50 kHz et environ 12 MHz.

5. Utilisation selon la revendication 4, dans laquelle la fréquence est située entre environ 1 MHz et environ 3 MHz.

6. Utilisation selon l'une quelconque des revendications 1 à 5, dans laquelle ledit composé d'hypocrelline amino-substituée peut, de plus, être activé par la lumière d'une longueur d'onde prédéterminée.

7. Utilisation selon l'une quelconque des revendications précédentes 1 à 6, dans laquelle ledit composé est une hypocrelline B déméthoxylée.

8. Utilisation selon la revendication 7, dans laquelle ladite hypocrelline B déméthoxylée est la 2-butylamino-2-déméthoxy-hypocrelline B.

9. Utilisation selon l'une quelconque des revendications 1 à 8 d'une composition comprenant ledit agent sonosensibilisant et au moins l'un d'un modificateur de pKa, un tampon, un sel, une base, un acide, une eau saline et un adjuvant.

10. Utilisation selon l'une quelconque des revendications 1 à 9 d'un composé d'hypocrelline amino-substituée pour la préparation d'une composition pharmaceutique pour le traitement par la dynamique sonore de conditions dermiques, cancer, maladies virales, maladies rétrovirales, maladies bactériennes, maladies auto-immunes ou maladies fongiques.

11. Utilisation d'un composé d'hypocrelline amino-substituée pour la préparation d'une composition pharmaceutique sono-activable pour le traitement par le son des conditions dermiques, cancer, maladies virales, maladies rétrovirales, maladies bactériennes, maladies auto-immunes et maladies fongiques.

Fig. 1

Fig. 2